# EUROPEAN PATENT SPECIFICATION

(11) **EP 3 140 763 B1**
(45) Date of publication and mention of the grant of the patent: **20.05.2020**
(21) Application number: 15789480.9
(22) Date of filing: 05.05.2015
(51) Int. Cl.: G16B 15/30, G16B 40/20, G16C 20/50

(54) **BINDING AFFINITY PREDICTION SYSTEM AND METHOD**
BINDUNGSAFFINITÄTSVORHERSAGESYSTEM UND -VERFAHREN
SYSTÈME DE PRÉDICTION D'AFFINITÉ DE LIAISON ET PROCÉDÉ ASSOCIÉ

(30) Priority: 05.05.2014 US 201461988510 P
(43) Date of publication of application: 15.03.2017
(73) Proprietor: Atomwise Inc., San Francisco, CA 94103 (US)
(72) Inventor: HEIFETS, Abraham Samuel, San Francisco, CA 94103 (US); WALLACH, Izhar, San Francisco, CA 94103 (US); DZAMBA, Michael, San Francisco, CA 94103 (US)
(74) Representative: Grund, Martin
(86) International application number: PCT/CA2015/000296
(87) International publication number: WO 2015/168774

(56) References cited:
- US-A1- 2002 090 631
- US-A1- 2013 280 238

## Description

### FIELD

This disclosure relates generally to bioinformatics systems, and more particularly to systems and methods for predicting binding affinity of one or more molecules.

### BACKGROUND

The ability to accurately predict a molecule's binding affinity to a protein is a fundamental tool in the discovery of new medicines and the elucidation of natural and engineered biological processes Unfortunately, in binding affinity prediction, the number of factors governing binding is extensive and their interactions are poorly understood.

The accuracy of the prediction is dependent on the features being considered by the predictive model. Some binding affinity prediction solutions are known, including those that incorporate machine learning features. Some prior art solutions use relatively simple prediction models that may not reflect the complexity of chemical and protein structures. Also, they may not reflect the many variables of interactions between molecules and proteins that affect binding affinity. For example, a given interaction between a molecule and a protein can be affected by the distance, angle, atom type, charge and polarization, and surrounding stabilizing or destabilizing environmental factors involved.

Prior art solutions use simple features such as, e.g., knowledge-based scoring functions that use pairs/groups of atoms, for example one atom from a molecule (such as a drug target) and one atom from a protein. These pairs are then analyzed, for example, by evaluating the distances between these pairs to derive probable molecule/protein binding atom pairs. Scores may be aggregated and these aggregated scores may be analyzed so as to determine molecule/protein binding affinity or "fit". Performance of the binding affinity prediction solution depends on the model used, and these are usually constructed and trained manually, which is time consuming.

Prior art binding affinity solutions generally include ones that (i) are knowledge-based, (ii) are empirical, or (iii) include force-field based scoring functions. Knowledge-based features typically consist of counts of the number of times pairs of atoms or functional groups are separated by a given distance in the biological complex. Because these features are simple (two points separated by a distance), they are incapable of capturing the complex set of influencing factors described above. Empirical scoring functions fit a set of relative importance weights to a small number (few dozen) of hand-engineered features, such as the number of rotatable bonds, hydrogen bond donor-acceptor pairs, aromatic stacks, electrostatics, steric complementarity or strain, or solvent-accessible hydrophobic area. The development of these features requires expert knowledge and extensive manual tuning, yet any such feature will necessarily be a limited approximation since, as discussed above, even expert chemists cannot consistently disentangle the forces governing molecular interactions. Force-field based scoring functions are designed to be computationally efficient, which requires approximations to theoretical results from gas phase predictions. For example, such systems ignore or crudely approximate the important mediation of field strength by solvent molecules.

US patent application publication 2002/0090631 A1 describes systems and methods for predicting the interactions of proteins with other proteins, nucleic acids and small molecules. A database containing protein sequences and information regarding protein interactions is used to teach the machine. US patent application publication 2013/0280238 A1 discloses processes for identification of binding sites on protein molecules such as epitopes.

For the reasons stated, prior art binding affinity prediction solutions may not be as accurate as is desirable.

There is a need for solutions that provide more accurate and/or more efficient binding affinity prediction, or at least provide an alternate way to provide binding affinity prediction.

### SUMMARY

In accordance with one aspect of the invention, there is provided a system for predicting binding affinity of one molecule to one target protein as defined in claim 1.

In accordance with another aspect of the invention, there is provided a computer-implemented method for predicting binding affinity of one molecule to one target protein as defined in claim 10.

In this respect, before explaining at least one embodiment of the invention in detail, it is to be understood that the invention is not limited in its application to the details of construction and to the arrangements of the components set forth in the following description or illustrated in the drawings. The invention is capable of other embodiments and of being practiced and carried out in various ways. Also, it is to be understood that the phraseology and terminology employed herein are for the purpose of description and should not be regarded as limiting.

### BRIEF DESCRIPTION OF THE DRAWINGS

In the drawings, embodiments of the invention are illustrated by way of example. It is to be expressly understood that the description and drawings are only for the purpose of illustration and as an aid to understanding, and are not intended as a definition of the limits of the invention.
**FIG. 1** is a block diagram illustrating an example system for predicting binding affinity, according to an embodiment.
**FIG. 2** is a workflow diagram showing high level steps for predicting binding affinity, according to an embodiment.
**FIG. 3** is a schematic view of a geometric representation of input features in the form of a three-dimensional grid of voxels, according to an embodiment.
**FIG. 4** is a schematic view of an example molecule in two different orientations, according to an embodiment.
**FIG. 5** and **FIG. 6** are views of two molecules encoded onto a two-dimensional grid visualization of voxels, according an embodiment.
**FIG. 7** is a view of the visualization of **FIG. 6**, in which the voxels have been numbered, according to an embodiment.
**FIG. 8** is a schematic view of geometric representation of input features in the form of coordinate locations of atom centers, according to an embodiment.
**FIG. 9** is schematic view of the coordinate locations of **FIG. 8** with a range of locations, according to an embodiment.
**FIG. 10** is a depiction of applying multiple function computation elements (g1, g2, ...) to the voxel inputs (x1,x2,... ,x100) and composing the function computation element outputs together using g(), according to an embodiment.
**FIG. 11** is a schematic diagram of hardware components of the system of **FIG. 1**, according to an embodiment.

### DETAILED DESCRIPTION

In one aspect, this disclosure provides a computer system that includes a novel and innovative binding affinity prediction system. The binding affinity prediction system may generate accurate predictions regarding the binding affinity of one or more molecules with one or more proteins.

In an embodiment, the binding affinity prediction system may be configured to analyze large volumes of biological data. For example the computer system may include or link to a database including, for example, tens of millions of data points. Depending on various factors, such as the data sources, the application, among others, the number of data points may vary.

To facilitate representation of these geometric features from biological data, the system may be configured to create a data representation, e.g., a data structure that virtualizes natural space.

In an embodiment, a unique and innovative data representation is provided that acts as a "container" for biological data, that has a fixed input size, so as to enable the application of deep learning techniques to discover binding affinity (including based on a broader set of binding parameters) between one or more molecules and one or more proteins.

In contrast to image classification systems, which may be based, for example, on the relative positions and sizes of patches of color, the binding affinity prediction system disclosed herein may be configured to recognize the positions and distances among groups of various kinds of atoms.

As an example, for biological data, in an embodiment, the system may be configured to represent the presence of every atom in a given voxel as a different number for that entry, e.g., if a carbon is in a voxel, a value of 6 is assigned to that voxel because the atomic number of carbon is 6. However, such an encoding could imply that atoms with close atomic numbers will behave similarly, which may not be particularly useful depending on the application. Further, element behavior may be more similar within groups (columns on the periodic table), and therefore such an encoding poses additional work for the deep learning system to decode.

In another embodiment, the system may be configured to encode the types in what may be referred to herein as a "one-hot" encoding: every atom type has a separate channel. While there are over 100 elements, most are not encountered in biology. However, even representing the most common biological elements (i.e., H, C, N, O, F, P, S, CI, Br, I, Li, Na, Mg, K, Ca, Mn, Fe, Co, Zn) may yield 18 channels or 10,483 * 18 = 188,694 inputs to the receptor field. The number of possible inputs may be even higher when other characteristics of the atoms (for example, partial charge, presence in ligand versus protein target, electronegativity, or SYBYL atom type) are additionally presented, necessitating more input channels to differentiate between otherwise-equivalent atoms.

The data representation is encoded with the biological data in a way that enables the expression of various structural relationships associated with molecules/proteins, for example. Deep learning methods are then applied to the data encoded to the data representation, potentially enabling the generation of analysis results that reflect the structural characteristics of the molecules/proteins. This approach yields more complex features than the features that are used by prior art binding prediction techniques.

Conveniently, embodiments disclosed herein do not require the creation of complex structural features that generally must be constructed and/or tuned manually.

Rather, in one aspect, the binding affinity prediction system implements "deep learning" methods to extract (e.g., automatically) a set of features, distilled from binding data.

More specifically, embodiments disclosed herein adapt various techniques, such as those used in computational audio and video processing, where deep learning methods may be used to predict binding affinity in connection with large sets of biological data, thereby providing more results that may be more useful and/or accurate than those in the prior art.

In an embodiment, the binding affinity prediction system may be configured to enable the analysis of significant volumes of data that determine the varied and complex chemical and protein structures involved in performing accurate molecule/protein binding affinity predictions.

Moreover, the binding affinity prediction system may provide an efficient and scalable mechanism for enabling the analysis of biological data in part on structural characteristics of associated molecules/proteins.

The computer system may be implemented to provide, or be integrated with, a number of different systems including (a) a bioinformatics system, or (b) a drug discovery system. Other systems may also incorporate features disclosed herein, including, for example (a) a personalized medicine system or (b) a medical record system that incorporates binding affinity prediction.

In an embodiment, the computer system comprises: one or more computers, the one or more computers being linked a binding affinity prediction utility, which when executed applies to a data set, including those received or derived from one or more databases of information (such as biological information), a binding affinity prediction function so as to generate one or more binding affinity predictions.

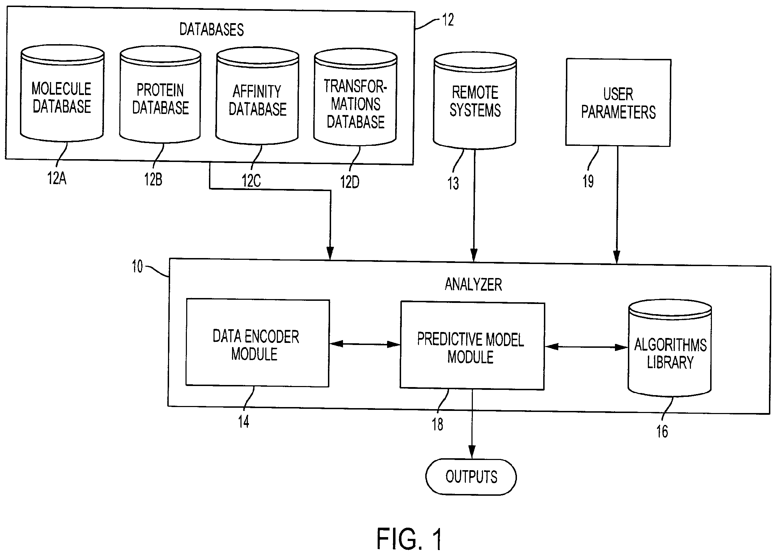

Referring to **FIG. 1**, a block schematic diagram is provided illustrating a sample implementation of the system, according to an embodiment.

As depicted, the system includes an analyzer **10**. The analyzer **10** may be linked to one or more databases **12** that store records reflecting input data (e.g., biological data). These databases, may include, databases such as, molecule database **12a**, protein database **12b**, affinity database **12c**, transformations database **12d**. The various databases are collectively referred to as databases **12**. Alternatively or in conjunction, the system may obtain biological data from one or more remote systems **13** that may also have additional biological data.

The molecule database **12a** and the protein database **12b** contain various biological, physical and/or chemical data, describing the relative positions of various atoms, ligand molecules, organic and inorganic cofactors, and proteins. For convenience, the term "biological data" may be used broadly herein to refer to all such data. The affinity database **12c** may include binding information, which may be numerical binding affinities. The transformations database **12d** may comprise various operands for the rotation, translation, and mirroring of geometries.

Data from the databases **12** and/or remote systems **13** may include data received as input data from a variety of sources, such as, e.g., structure ensembles generated by solution NMR, co-complexes as interpreted from X-ray crystallography, sampling from computational simulations, approximate pose-generation techniques such as homology modeling or rotamer library sampling, and combinations of these techniques. The input data may include both training examples and labels.

The analyzer **10** may include a data encoder module **14** and a predictive model module **18**. The analyzer **10** is also linked to one or more algorithm libraries **16** that include one or more learning algorithms for discovering or predicting likely binding affinities. The analyzer **10** receives inputs from the various databases **12** and/or remote systems **13** and provides them to the data encoder module **14** that encodes selected or defined biological data to one or more geometric data representations. The one or more geometric data representations are then provided to the predictive model module **18**, which applies one or more algorithms from the one or more algorithm libraries to determine predictions on likely binding affinities.

The predictive model module **18** may be configured to utilize various deep learning techniques and/or machine learning techniques, which are used for prediction.

The predictive model module **18** may further be configured to be trained over time as more inputs are processed. In an embodiment, the predictive model module **18** is configured to utilize neural networks with weighted connections, and may further be configured to adapt weightings based upon various training events and/or conditions. The algorithm libraries **16** may be configured to support training by storing and/or processing information related to prior operation of the predictive model module **18**, which may include information such as connection weighting, etc.

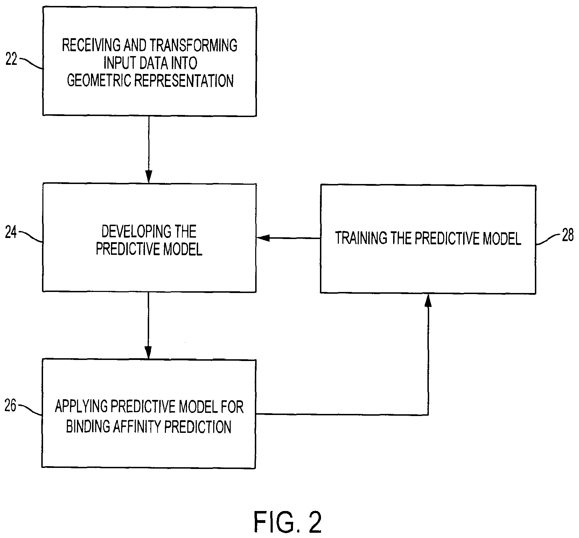

Referring to **FIG. 2**, a workflow is provided indicating high level steps for predicting binding affinity, according to an embodiment.

The workflow contains the steps of receiving and transforming input data into geometric representation **22**, developing the predictive model **24**, applying the predictive model for binding affinity prediction **26** and training the predictive model **28**. It will be understood that the steps are provided solely for illustrative purposes and depending on the embodiment, there may be more, or fewer steps, and the steps may vary, or may be in other orders.

These steps are described in more detail below.

### Receiving and encoding input data into a geometric representation

In the receiving and encoding input data into geometric representation step **22**, the analyzer **10** may receive a number of inputs from databases **12** and/or remote systems **13** and develops, using the data encoder module **14**, a data representation (e.g., a data structure) that creates a geometric representation, which in an embodiment, is virtualized real space.

Data from databases **12** and/or remote systems **13** may describe various biological, physical and/or chemical data, describing the relative positions of various atoms, ligand molecules, organic and inorganic cofactors, and proteins. The data is collectively described as "input data".

The data encoder module **14** is configured to construct a data structure providing a geometric representation of binding input features, and to populate the data structure by encoding data relating to one or more molecules and more or more target proteins. The data for encoding may be selected from the above-noted input data.

Input geometric data may be grouped into training examples. For example, it is often the case that a single set of molecules, cofactors, and proteins has multiple geometric measurements, where each "snapshot" describes alternative conformations and poses that the protein and molecules may adopt. Similarly, different tautomers for the protein sidechains, cofactors, and ligands may also be sampled. Because these states all contribute to the behavior of the biological system, as per the Boltzmann distribution, a system to predict binding affinity may be configured to consider these states together.

Optionally, these training examples may be labelled with binding information. If quantitative binding information is available, the labels may be the numerical binding affinities. Alternatively, the training examples may be assigned labels from a set of two or more ordered categories (e.g., two categories of binders and nonbinders, or several possibly-overlapping categories describing the ligands as binders of potencies <1 molar, <1 millimolar, <100 micromolar, <10 micromolar, <1 micromolar, <100 nanomolar, <10 nanomolar, <1 nanomolar). Binding information may be derived or received from a variety of sources, such as experimental measurements, computed estimates, expert insight, or presumption (for example, a random pair of molecule and protein are highly unlikely to bind).

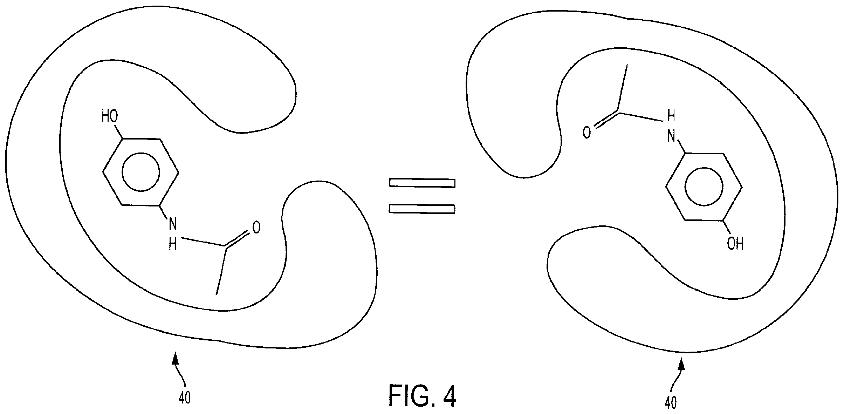

The input data is processed. The data encoder module **14** is further configured to augment the input data by generating additional instances through the application of one or more pre-defined transformation, e.g., including some combination of rotation, translation, and mirroring operators in any combination of the three X, Y and Z planes. Rotation and translation of the geometric data may be randomly selected (within some range, e.g. plus or minus 5Å from the origin) or uniformly generated at some pre-specified increment (e.g., all 5 degree increments around the circle), or adversarially chosen (e.g., to generate incorrect predictions from a given predictive system). This augmentation of the data may be configured through inputs from the transformations database **12d**, which may be comprised of one or more data augmentation operators. **FIG. 4** provides a sample illustration of an example molecule **40** in two different orientations.

After the data augmentation operators (if any) are applied, the data encoder module **14** is configured to truncate the resulting geometric data. Because neural networks require a fixed input size, the system may crop the geometric data to fit within an appropriate bounding box. For example, a cube of 25 - 40Å to a side, may be used. The input data may also be translated (e.g., mapped) into a fixed-size grid.

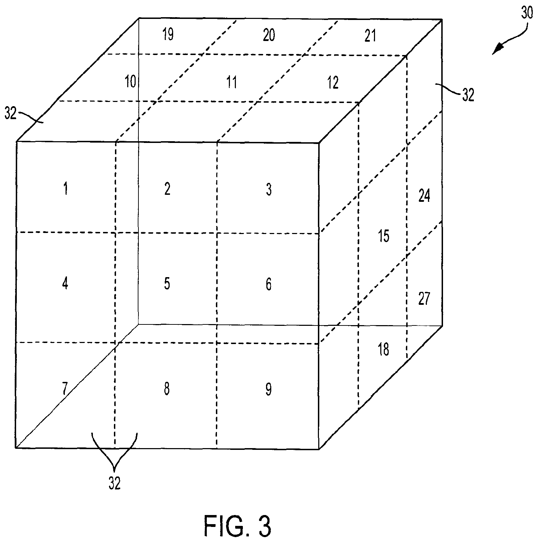

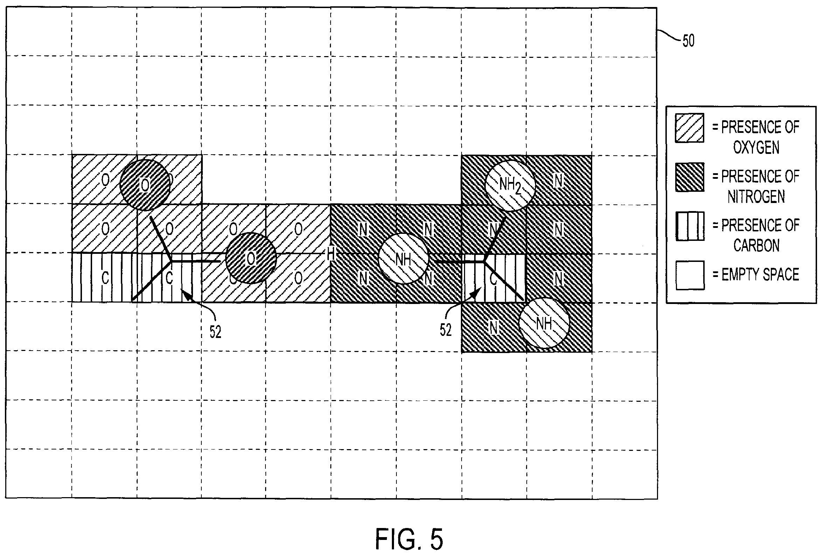

The geometric representation may be implemented in a variety of ways and topographies, according to various embodiments. The geometric representation is used for the visualization and analysis of data. For example, in an embodiment, geometries may be represented using voxels laid out on various topographies, such as 2-D, 3-D Cartesian / Euclidean space, 3-D non-Euclidean space, manifolds, etc. For example, **FIG. 3** provides an example representation comprising a three-dimensional grid of voxels, and **FIG. 5** provides an example representation comprising a two-dimensional grid of voxels.

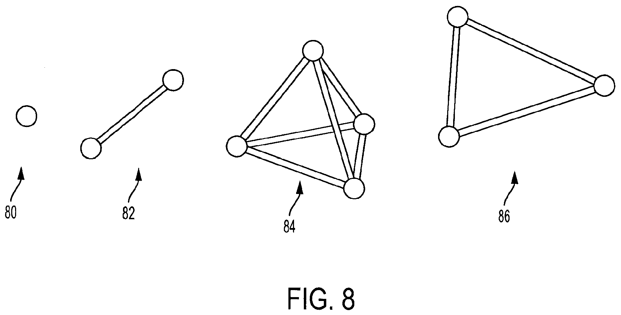

In another embodiment, the geometry may be represented by a set of points in 3-D space with associated pairwise distance annotations, e.g., as shown in **FIG. 8**.

The voxels may have particular values associated to them, which may, for example, be represented by applying labels, and/or determining their positioning, among others.

The data encoder module **14** may be configured to use any of a variety of shapes to partition the space into the voxel grid. In some embodiments, polyhedra, such as rectangles, polygonal shapes, etc. may be used to partition the space.

In an embodiment, the grid structure may be configured to be similar to an arrangement of voxels. For example, each sub-structure may be associated with a channel for each atom being analyzed. Also, an encoding method may be provided for representing each atom numerically.

In an embodiment, the data encoder module **14** may be configured to transform the geometric data into a discrete set of regularly-spaced Euclidean grid of cubic voxels. The data encoder module **14** may be configured to set geometric shapes at a variety of different resolutions. For example, a resolution of 1Å may be chosen, but the system could also be configured to choose finer (e.g., 0.1Å or even 0.01Å) or coarser resolutions (e.g. 4Å or 4Å), where the spacing yields an integer number of cubes to cover the input data.

As an illustration, for a 40Å input cube, with a 1Å resolution, such an arrangement would yield 40 * 40 * 40 = 64,000 input voxels.

In some embodiments, the geometric representation is generated, taking into account the factor of time and may thus be in four dimensions (X, Y, Z, and time).

In some embodiments, other implementations such as pixels, points, polygonal shapes, polyhedrals, or any other type of shape in multiple dimensions (e.g. shapes in 3-D, 4-D, and so on) may be used instead of voxels.

In an embodiment, the geometric information may be represented by the X,Y,Z coordinates of the centers of the atoms comprising the ligand, co-factors, and the protein. One alternative representation is the electron density as measured, for example, in X-ray crystallography.

In an embodiment, every voxel has one or more input channels, which may have various values associated with them, which in a simple implementation could be on/off, and may be configured to encode for a type of atom. Atom types may denote the element of the atom, or atom types may be further refined to distinguish between other atom characteristics.

In an embodiment, the system may be configured to normalize the geometric data by choosing the origin of the X,Y, Z coordinates to be the center of mass of the binding site as determined by a cavity flooding algorithm. Alternatively, the system could be configured to center the origin at the center of mass of the entire co-complex, of just the protein, or of just the ligand. The basis vectors may optionally be chosen to be the principal moments of inertia of the entire co-complex, of just the protein, or of just the ligand.

Atoms present may then be encoded in each voxel. Various types of encoding may be utilized using various techniques and/or methodologies. As an example encoding method, the atomic number of the atom may be utilized, yielding one value per voxel ranging from one for hydrogen to 118 for ununoctium (or any other element).

However, other encoding methods may be utilized, such as "one-hot encoding" noted above, where, every voxel has many parallel input channels, each of which is either on or off and encodes for a type of atom, as shown in **FIGS. 5****,** **6****,** and **7****.** Atom types may denote the element of the atom, or atom types may be further refined to distinguish between other atom characteristics. For example, Sybyl atom types distinguish single-bonded carbons from double-bonded, triple-bonded, or aromatic carbons.

In an embodiment, the data encoder module **14** further distinguishes between atoms that are part of the protein or cofactors versus part of the ligand.

Other channels may additionally (or alternatively) specify further information such as partial charge, polarizability, electronegativity, solvent accessible space, and electron density.

In some embodiments, the data encoder module **14** may also be configured for other additional optional encoding refinements. The following are provided as examples.

Most elements rarely occur in biological systems. In a first encoding refinement, the required memory may be reduced by reducing the set of atoms represented by the system. Atoms may be mapped to share the same channel, either by combining rare atoms (which may therefore rarely impact the performance of the system) or by combining atoms with similar properties (which therefore could minimize the inaccuracy from the combination).

A second encoding refinement may be to configure the system to represent atom positions by partially activating neighboring voxels. Partial activation of neighboring neurons moves away from one-hot encoding to a "several-warm" encoding. For example, it may be illustrative to consider a chlorine atom, which has a van der Waals diameter of 3.5Å and therefore a volume of 22.4Å³ when a 1Å³ grid is placed, voxels inside the chlorine atom will be completely filled and voxels on the edge of the atom will only be partially filled. The system may be configured to partially turn on the partially-filled voxels proportionately to the volume of the voxel which is inside the chlorine atom. This may result in a "smoothed" and more accurate representation relative to the discrete one-hot encoding.

The output from the data encoder module **14** is a set of geometric data that has been encoded based upon various rules applied to the input data.

**FIG. 3** illustrates a sample three-dimensional grid structure **30** including a series of sub-containers **32**, according to an embodiment. Each sub-container **32** may correspond to a voxel. A coordinate system may be defined for the grid, such that each sub-container has an identifier. In the depicted embodiment, the coordinate system is a Cartesian system in 3-D space. However, in other embodiments, the coordinate system may be any other type of coordinate system, such as an oblate spheroid, cylindrical or spherical coordinate systems, polar coordinates systems, other coordinate systems designed for various manifolds and vector spaces, among others.

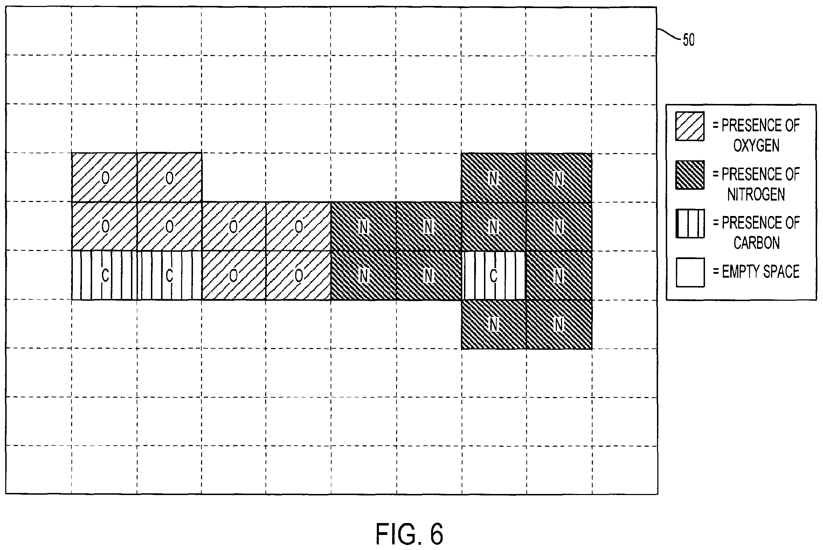

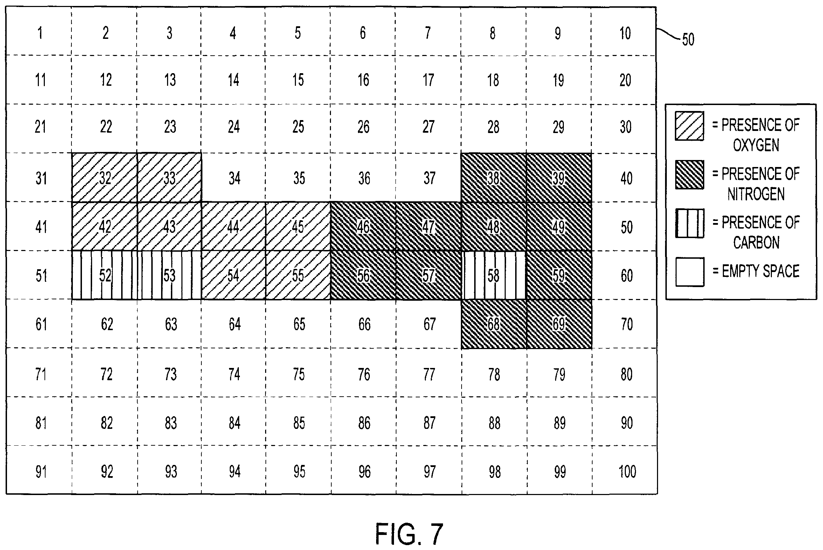

**FIG. 5** and **FIG. 6** each provide views of two molecules encoded onto a two-dimensional grid **50** of voxels, according to an embodiment. **FIG. 5** provides the two molecules **52** superimposed on the two-dimensional grid **50**. As shown, each position in the grid is encoded with an identifier, e.g., identifying the presence of oxygen, nitrogen, carbon, and empty space. As noted above, such encoding may be referred to as "one-hot" encoding. **FIG. 6** shows the grid **50** of **FIG. 5** with the molecules **52** omitted. **FIG. 7** provides a view of the grid **50**, in which each voxel has been numbered.

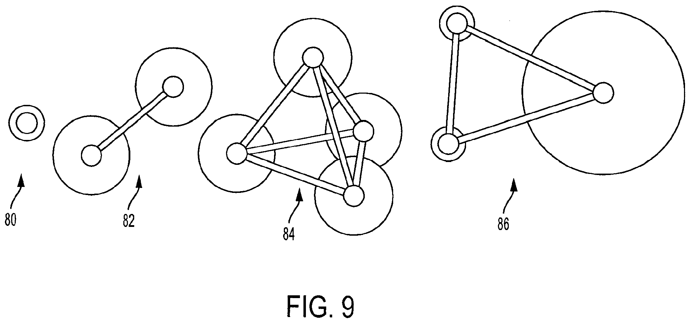

As noted, feature geometry may be represented in forms other than voxels. **FIG. 8** provides views of various representations in which features (e.g., atom centers) are represented as 0-D points (representation **80**), 1-D points (representation **82**), 2-D points (representation **86**), or 3-D points (representation **84**). Initially, the spacing between the points may be randomly chosen. However, as the predictive model is trained, the points may be moved closer together, or father apart. **FIG. 9** illustrates a range of possible positions for each point.

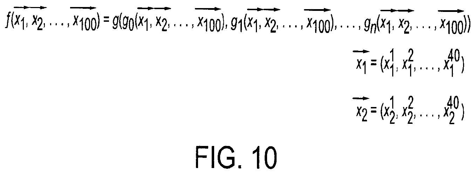

**FIG. 10** provides a depiction of applying multiple function computation elements (g1, g2, ...) to the voxel inputs (x1,x2,...,x100) and composing the function computation element outputs together using g(), according to some embodiments. For example, each voxel input may be a one-hot encoded vector of 40 distinct atom types.

### Developing the Predictive Model

The predictive model module **18** is configured to utilize deep learning methods to use the geometric data output from the data encoder module **14** to develop a predictive model in step **24**. The deep learning methods may be supervised, or unsupervised methods, according to various embodiments.

The predictive model module **18** may be configured to utilize various deep learning methods, which may include, as illustrative, non-limiting examples, deep neural networks, convolutional neural networks, deep belief networks, stacked Boltzmann machines, autoencoders, sparse codes, and topological principle component analysis, among others.

In an embodiment, the predictive model module **18** may be configured to select and apply one or more deep learning methods from the one or more algorithm libraries **16** based upon user defined parameters **19**. These user defined parameters 19 may further configure the predictive model module **18** to apply the one or more deep learning methods at various settings. These settings may allow the predictive model module **18** to be run on a number of settings, each giving different tradeoffs of security, speed, and flexibility.

Training data may be provided to the predictive model module **18** to build and train the predictive model over a period of time.

In order to apply deep learning methods, it may be a requirement to have a fixed input size to the binding affinity prediction system. Also, accurate discovery prediction of binding affinity may depend on geometric features of the molecules/proteins.

The deep learning methods may be used to extract one or more features that may be important for an analysis of binding affinity. A potential advantage of utilizing deep learning methods is that relationships that may not be intuitively clear or operable by humans can be identified and used.

These features may be composited by the predictive model module **18** into general discriminatory filters. In an embodiment, deep learning techniques may be adapted to be applied to data to describe the geometric relationship between, for example, a ligand and a protein.

Accordingly, the operation of the predictive model module **18** may yield more complex features than the features historically used to conduct binding affinity prediction. For example, a feature that serves as a hydrogen bond detector may be able to recognize not only that a hydrogen bond donor and acceptor are at a given distance and at given angles, but also recognize that the biochemical environment around the donor and acceptor strengthens or weakens the bond. Additionally, the detectors that the system derives may be those that effectively discriminate binders from non-binders in the underlying data.

In an embodiment, the predictive model module **18** may be configured to adapt for dynamic biological systems, such as, the alternative positions that may be encountered as both the protein and the ligand move. In such a protein-ligand complex, a number of different configurations may be adopted, with the relative proportion based on the Boltzmann distribution of the free energy of each shape. Both the enthalpic and entropic components of the free energy of the protein-ligand complex can depend on the poses adopted by the molecules (ΔG = ΔH - T ΔS). The final binding affinity may be found to be a function of the weighted average of the energies of the set of poses available to the complex. To model this physical phenomenon, the predictive model module **18** may be configured to sample a large number of alternative positions due to ligand and protein motion and to base its binding affinity predictions on this sampled set of configurations of the complex.

In an embodiment, where a deep neural network is implemented, the predictive model module **18** may be configured to train the deep neural network to receive the geometric data input and to output a prediction (probability) of whether or not a given ligand binds to a protein target. The deep neural network may be comprised of a set of functional computation elements. These function computation elements may be feature detectors. In an embodiment, the system may be configured to utilize function computation elements which scale each of their inputs by a weight parameter, sum the result, add a bias, and apply an activation function to the result.

Examples of activation functions for the neural network may include, but are not limited to, logistic (or sigmoid), softmax, Gaussian, Boltzmann-weighted averaging, absolute value, linear, rectified linear, bounded rectified linear, soft rectified linear, parameterized rectified linear, average, max, min, some vector norm LP (for p=1,2,3,...,∞), sign, square, square root, multiquadric, inverse quadratic, inverse multiquadric, polyharmonic spline, and thin plate spline.

The inputs to the function computation elements can be the activations in the voxel channels of the receptive field.

The functions may also be cascaded, where the outputs of some function computation elements serve as inputs to other function computation elements. A subset of function computation elements that take the same inputs may be referred to as a "layer".

Zero or more of the layers may consist of convolutional filters. A convolutional filter layer (or "feature map") is a set of function computation elements, each of which takes as input a region of spatially contiguous voxels or convolutional functions.

The set may be selected to cover the receptive field. The parameter weights (and, optionally, biases) of every function in a given convolutional layer may be tied together, i.e. constrained to be identical. Because the functions compute the same output at different locations of the receptive field, the convolutional layer may be able to detect local input patterns even after translation of X, Y and Z coordinates.

In an embodiment, the predictive model module **18** may be configured to develop three-dimensional convolutional layers. The input region to the lowest level convolutional layer may be a cube (or other contiguous region) of voxel channels from the receptive field. Higher convolutional layers may also consider the output from lower convolutional layers, while still having their output be a function of a bounded region of voxels which are close together (in 3-D Euclidean distance).

Biological activity may be invariant under rotation, as well as translation, so the predictive model module **18** may be optionally configured to generate rotated feature maps that take advantage of the rotational symmetries of space partitioning. For example, if the system was configured to use cubes to partition the input data, the system could be configured to generate rotated feature maps by tying the weights of the function computations together after a 90 degree rotation.

It may be illustrative to consider a cube which is rotated clockwise: the weights in the upper face of one filter become tied to the weights in the right face of a different filter; in other words, the weights may be constrained to be identical. Rotation may generate 24 feature maps by rotating clockwise by 90 degrees, 180 degrees, 270 degrees, for each of the three XY / XZ / YZ planes. This arrangement reduces the number of parameters to 1/24th of without rotational weight tying, since without weight tying every filter has its own weights.

As an alternative example, if the system were configured to use other polyhedra to partition the input data, the system may be configured to use other rotations to access the isometries appropriate to their symmetry groups. For example, where space has been partitioned using truncated octahedrons, there would be 3 axes of 90 degree rotational symmetry, 4 axes of 120 degree rotational symmetry, and six axes of 180 degree symmetry.

In an embodiment, the predictive model module **18** may be configured to apply regularization techniques to remove artifacts from the model, which may reduce the complexity and processing load required reduce the effect of orientation on the analysis of the model, reducing the importance of elements being in particular positions.

Zero or more of the neural network layers may consist of pooling layers. As in a convolutional layer, a pooling layer is a set of function computations that apply the same function over different spatially-local patches of input. For pooling layers, the output is given by a pooling operators, e.g. some vector norm LP for p=1,2,3,...,∞, over several voxels. Pooling is typically done per channel, rather than across channels.

Zero or more of the layers may consist of normalization layers, such as local response normalization or local contrast normalization, which may be applied across channels at the same position or for a particular channel across several positions. These normalization layers may encourage variety in the response of several function computations to the same input.

In an embodiment, the predictive model module **18** may be configured so that the output of a subset of the function computations may be provided into a classification system. A fully connected single layer or multilayer perceptron may be applied, but other classifiers such as support vector machine, random forest, nearest neighbor, etc. could also be applied. Preferably, the classifier assigns a numeric score indicating the strength (or confidence or probability) of classifying the input into the various output categories. In some cases, the categories are binders and nonbinders or, alternatively, the potency level (potencies <1 molar, <1 millimolar, <100 micromolar, <10 micromolar, <1 micromolar, <100 nanomolar, <10 nanomolar, <1 nanomolar).

The geometric data poses may have been grouped into training examples. To present all instances in an example at once may require a prohibitively large input field (i.e., an input field of size equal to number of voxels * number of channels * number of poses).

As a potential solution, each instance in the example may be presented separately to the neural network and the classification scores of each instance may be combined together to produce a final score for the entire example.

In an embodiment, where the classifier output is numeric, the outputs may be combined using any of the activation functions described above.

In an embodiment, where the classifier output is not numeric, the predictive model module **18** may be configured to combine the outputs using utilize various ensemble voting schemes, which may include, as illustrative, non-limiting examples, majority, weighted averaging, Condorcet methods, Borda count, among others.

In an embodiment, the system may be configured to apply an ensemble of predictive models, e.g., to generate indicators of binding affinity.

### Applying the Predictive Model for Binding Affinity Prediction

Upon developing the predictive model within the predictive model module **18**, the analyzer **10** may then receive inputs from one or more databases **12** and/or one or more remote systems **13** one or more data sets describing a protein target to be selected for analysis with the predictive model. The inputs from the databases **12** may include a set of molecules to be analyzed.

The analyzer **10** may then apply the predictive model to the information received and the analyzer **10** may return one or more outputs. The outputs provided from the system may vary, and may range from numerical scores to lists of molecules selected from the set of molecules to be analyzed which have a score greater than a pre-determined threshold. The output may be an indicator of binding affinity for one or more molecules to one or more target proteins (or types of protein), e.g., a score and/or a probability of molecule being active or not active given a specific protein or type of protein.

In an embodiment, the final interpretation of scores can either be based on the numerical score alone or the position of the score of the molecule in a ranked list relative to the scores of predetermined active and inactive molecules.

### Training the Predictive Model

The deep learning techniques may have a training step **28** to improve the accuracy of prediction over time.

In an embodiment, where the deep learning techniques utilizes a a neural network as described above, the predictive model module **18** may be configured to train the predictive model to improve the accuracy of its prediction by modifying the weights and biases in the function computations. Historical weights and prediction accuracy may be stored and/or accessed from the algorithm libraries **16**. The parameters may be further constrained with various forms of regularization such as L1, L2, weight decay, and dropout.

In an embodiment, the predictive model module **18** may be configured to tune the weights to model the input distribution of the training data through greedy, layerwise, generative pre-training using the contrastive divergence algorithm.

In an embodiment, the predictive model module **18** may, where training data is labelled, tune the weights to potentially minimize the error between the model's predicted binding affinities and/or categorizations and the training data's reported binding affinities and/or categorizations. Various methods may be used to minimize error function, such as gradient descent methods, which may include, but are not limited to, log-loss, sum of squares error, hinge-loss methods. These methods may include second-order methods or approximations such as momentum, Hessian-free estimation, Nesterov's accelerated gradient, adagrad, etc. Unlabelled generative pretraining and labelled discriminative training may also be combined.

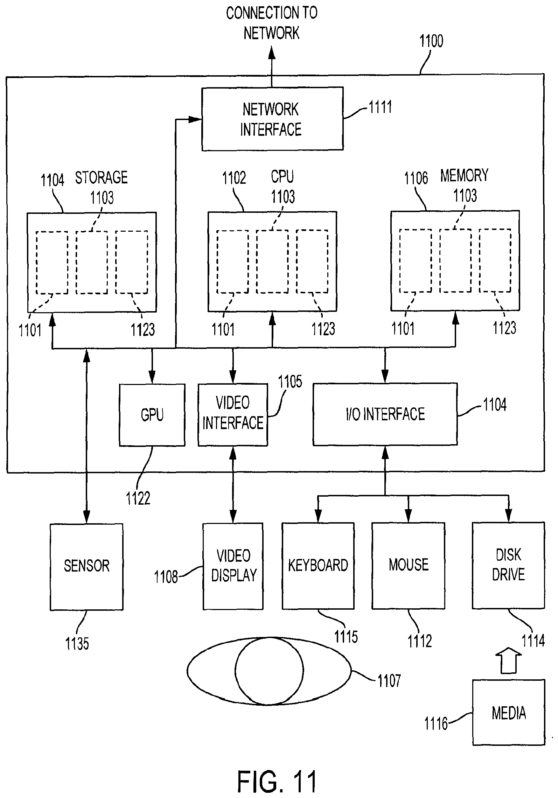

The present system and method may be practiced in various embodiments. A suitably configured computer device, and associated communications networks, devices, software and firmware may provide a platform for enabling one or more embodiments as described above. By way of example, **FIG. 11** shows a example computer device **1100** that may include a central processing unit ("CPU") **1102** connected to a storage unit **1104** and to a random access memory **1106.** The CPU **1102** may process an operating system **1101,** application program **1103,** and data **1123**. The operating system **1101,** application program **1103**, and data **1123** may be stored in storage unit **1104** and loaded into memory **1106**, as may be required. Computer device **1100** may further include a graphics processing unit (GPU) **1122** which is operatively connected to CPU **1102** and to memory **1106** to offload intensive data processing calculations from CPU **1102** and run these calculations in parallel with CPU **1102**. An operator **1107** may interact with the computer device **1100** using a video display **1108** connected by a video interface **1105**, and various input/output devices such as a keyboard **1115**, mouse **1112**, and disk drive or solid state drive **1114** connected by an I/O interface **1109**. In known manners, the mouse **1112** may be configured to control movement of a cursor in the video display **1108**, and to operate various graphical user interface (GUI) controls appearing in the video display **1108** with a mouse button. The disk drive or solid state drive **1114** may be configured to accept computer readable media **1116**. The computer device **1100** may form part of a network via a network interface **1111**, allowing the computer device **1100** to communicate with other suitably configured data processing systems (not shown). One or more different types of sensors **1135** may be used to receive input from various sources.

The present system and method may be practiced on virtually any manner of computer device including a desktop computer, laptop computer, tablet computer or wireless handheld. The present system and method may also be implemented as a computer-readable/useable medium that includes computer program code to enable one or more computer devices to implement each of the various process steps disclosed herein. In case of more than computer devices performing the entire operation, the computer devices are networked to distribute the various steps of the operation. It is understood that the terms computer-readable medium or computer useable medium comprises one or more of any type of physical embodiment of the program code. In particular, the computer-readable/useable medium can comprise program code embodied on one or more portable storage articles of manufacture (e.g. an optical disc, a magnetic disk, a tape, etc.), on one or more data storage portioned of a computing device, such as memory associated with a computer and/or a storage system.

The functionality described may be implemented to any mobile platform, including the iOS™ platform, ANDROID™, WINDOWS™ or BLACKBERRY™.

### Example Use Cases

The following are sample use cases provided for illustrative purposes only that describe some applications of some embodiments. Other uses may be considered, and the examples provided below are non-limiting and may be subject to variations, omissions, or may contain additional elements.

While each example below illustrates binding affinity prediction, the examples may be found to differ in whether the predictions are made over a single molecule, a set, or a series of iteratively modified molecules; whether the predictions are made for a single target or many, whether activity against the targets is to be desired or avoided, and whether the important quantity is absolute or relative activity; or, if the molecules or targets sets are specifically chosen (e.g., for molecules, to be existing drugs or pesticides; for proteins, to have known toxicities or side-effects).

**Hit discovery**: Pharmaceutical companies spend millions of dollars on screening compounds to discover new prospective drug leads. Large compound collections are tested to find the small number of compounds that have any interaction with the disease target of interest. Unfortunately, wet lab screening suffers experimental errors and, in addition to the cost and time to perform the assay experiments, the gathering of large screening collections imposes significant challenges through storage constraints, shelf stability, or chemical cost. Even the largest pharmaceutical companies have only between hundreds of thousands to a few millions of compounds, versus the tens of millions of commercially available molecules and the hundreds of millions of simulate-able molecules.

A potentially more efficient alternative to physical experimentation is virtual high throughput screening. In the same manner that physics simulations can help an aerospace engineer to evaluate possible wing designs before a model is physically tested, computational screening of molecules can focus the experimental testing on a small subset of high-likelihood molecules. This may reduce screening cost and time, reduces false negatives, improves success rates, and/or covers a broader swath of chemical space.

In this application, a protein target may be provided as input to the system. A large set of molecules may also be provided. For each molecule, a binding affinity is predicted against the protein target. The resulting scores may be used to rank the molecules, with the best-scoring molecules being most likely to bind the target protein. Optionally, the ranked molecule list may be analyzed for clusters of similar molecules; a large cluster may be used as a stronger prediction of molecule binding, or molecules may be selected across clusters to ensure diversity in the confirmatory experiments.

**Off-target side-effect prediction:** Many drugs may be found to have side-effects. Often, these side-effects are due to interactions with biological pathways other than the one responsible for the drug's therapeutic effect. These off-target side-effects may be uncomfortable or hazardous and restrict the patient population in which the drug's use is safe. Off-target side effects are therefore an important criterion with which to evaluate which drug candidates to further develop. While it is important to characterize the interactions of a drug with many alternative biological targets, such tests can be expensive and time-consuming to develop and run. Computational prediction can make this process more efficient.

In applying an embodiment, a panel of biological targets may be constructed that are associated with significant biological responses and/or side-effects. The system may then be configured to predict binding against each protein in the panel in turn. Strong activity (that is, activity as potent as compounds that are known to activate the off-target protein) against a particular target may implicate the molecule in side-effects due to off-target effects.

**Toxicity prediction:** Toxicity prediction is a particularly-important special case of off-target side-effect prediction. Approximately half of drug candidates in late stage clinical trials fail due to unacceptable toxicity. As part of the new drug approval process (and before a drug candidate can be tested in humans), the FDA requires toxicity testing data against a set of targets including the cytochrome P450 liver enzymes (inhibition of which can lead to toxicity from drug-drug interactions) or the hERG channel (binding of which can lead to QT prolongation leading to ventricular arrhythmias and other adverse cardiac effects).

In toxicity prediction, the system may be configured to constrain the off-target proteins to be key antitargets (e.g. CYP450, hERG, or 5-HT_{2B} receptor). The binding affinity for a drug candidate may then predicted against these proteins. Optionally, the molecule may be analyzed to predict a set of metabolites (subsequent molecules generated by the body during metabolism/degradation of the original molecule), which can also be analyzed for binding against the antitargets. Problematic molecules may be identified and modified to avoid the toxicity or development on the molecular series may be halted to avoid wasting additional resources.

**Potency optimization:** One of the key requirements of a drug candidate is strong binding against its disease target. It is rare that a screen will find compounds that bind strongly enough to be clinically effective. Therefore, initial compounds seed a long process of optimization, where medicinal chemists iteratively modify the molecular structure to propose new molecules with increased strength of target binding. Each new molecule is synthesized and tested, to determine whether the changes successfully improved binding. The system may be configured to facilitate this process by replacing physical testing with computational prediction.

In this application, the disease target and a set of lead molecules may be input into the system. The system may be configured to produce binding affinity predictions for the set of leads. Optionally, the system could highlight differences between the candidate molecules that could help inform the reasons for the predicted differences in binding affinity. The medicinal chemist user can use this information to propose a new set of molecules with, hopefully, improved activity against the target. These new alternative molecules may be analyzed in the same manner.

**Selectivity optimization:** As discussed above, molecules tend to bind a host of proteins at a variety of strengths. For example, the binding pockets of protein kinases (which are popular chemotherapy targets) are very similar and most kinase inhibitors affect many different kinases. This means that various biological pathways are simultaneously modified, which yields a "dirty" medicinal profile and many side-effects. The critical challenge in the design of many drugs, therefore, is not activity *per* se but specificity: the ability to selectively target one protein (or a subset of proteins) out from a set of possibly-closely related proteins.

Our system can reduce the time and cost of optimizing the selectivity of a candidate drug. In this application, a user may input two sets of proteins. One set describes proteins against which the compound should be active, while the other set describes proteins against which the compound should be inactive. The system may be configured to make predictions for the molecule against all of the proteins in both sets, establishing a profile of interaction strengths. Optionally, these profiles could be analyzed to suggest explanatory patterns in the proteins. The user can use the information generated by the system to consider structural modifications to a molecule that would improve the relative binding to the different protein sets, and to design new candidate molecules with better specificity. Optionally, the system could be configured to highlight differences between the candidate molecules that could help inform the reasons for the predicted differences in selectivity. The proposed candidates can be analyzed iteratively, to further refine the specificity of their activity profiles.

**Fitness function for automated molecular design:** Automated tools to perform the preceding optimizations are valuable. A successful molecule requires optimization and balance among potency, selectivity, and toxicity. "Scaffold hopping" (when the activity of a lead compound is preserved but the chemical structure is significantly altered) can yield improved pharmacokinetics, pharmacodynamics, toxicity, or intellectual property profiles. Algorithms exist to iteratively suggest new molecules, such as random generation of molecules, growth of molecular fragments to fill a given binding site, genetic algorithms to "mutate" and "cross-breed" a population of molecules, and swapping of pieces of a molecule with bioisosteric replacements. The drug candidates generated by each of these methods must be evaluated against the multiple objectives described above (potency, selectivity, toxicity) and, in the same way that the technology can be informative on each of the preceding manual settings (binding prediction, selectivity, side-effect and toxicity prediction), it can be incorporated in an automated molecular design system.

**Drug repurposing:** All drugs have side-effects and, from time to time, these side-effects are beneficial. The best known example might be aspirin, which is generally used as a headache treatment but is also taken for cardiovascular health. Drug repositioning can significantly reduce the cost, time, and risk of drug discovery because the drugs have already been shown to be safe in humans and have been optimized for rapid absorption and favorable stability in patients. Unfortunately, drug repositioning has been largely serendipitous. For example, sildenafil (Viagra), was developed as a hypertension drug and was unexpectedly observed to be an effective treatment for erectile dysfunction. Computational prediction of off-target effects can be used in the context of drug repurposing to identify compounds that could be used to treat alternative diseases.

In this application, as in off-target side-effect prediction, the user may assemble a set of possible target proteins, where each protein is linked to a disease. That is, inhibition of each protein would treat a (possibly different) disease; for example, inhibitors of Cyclooxygenase-2 can provide relief from inflammation, whereas inhibitors of Factor Xa can be used as anticoagulants. These proteins are annotated with the binding affinity of approved drugs, if any exist. We then assemble a set of molecules, restricting the set to molecules that have been approved or investigated for use in humans. Finally, for each pair of protein and molecule, the user may use the system to predict the binding affinity. Candidates for drug repurposing may be identified if the predicted binding affinity of the molecule is close to the binding affinity of effective drugs for the protein.

**Drug resistance prediction:** Drug resistance is an inevitable outcome of pharmaceutical use, which puts selection pressure on rapidly dividing and mutating pathogen populations. Drug resistance is seen in such diverse disease agents as viruses (HIV), exogenous microorganisms (MRSA), and disregulated host cells (cancers). Over time, a given medicine will become ineffective, irrespective of whether the medicine is antibiotics or chemotherapies. At that point, the intervention can shift to a different medicine that is, hopefully, still potent. In HIV, there are well-known disease progression pathways that are defined by which mutations the virus will accumulate while the patient is being treated.

There is considerable interest in *predicting* how disease agents adapt to medical intervention. One approach is to characterize which mutations will occur in the disease agent while under treatment. Specifically, the protein target of a medicine needs to mutate so as to avoid binding the drug while simultaneously continue to bind its natural substrate.

In this application, a set of possible mutations in the target protein may be proposed. For each mutation, the resulting protein shape may be predicted. For each of these mutant protein forms, the system may be configured to predict a binding affinity for both the natural substrate and the drug. The mutations that cause the protein to no longer bind to the drug but also to continue binding to the natural substrate are candidates for conferring drug resistance. These mutated proteins may be used as targets against which to design drugs, e.g. by using these proteins as inputs to one of these other prediction use cases.

**Personalized medicine:** Ineffective medicines should not be administered. In addition to the cost and hassle, all medicines have side-effects. Moral and economic considerations make it imperative to give medicines only when the benefits outweigh these harms. It may be important to be able to predict when a medicine will be useful. People differ from one another by a handful of mutations. However, small mutations may have profound effects. When these mutations occur in the disease target's active (orthosteric) or regulatory (allosteric) sites, they can prevent the drug from binding and, therefore, block the activity of the medicine. When a particular person's protein structure is known (or predicted), the system can be configured to predict whether a drug will be effective or the system may be configured to predict when the drug will *not work.*

For this application, the system may be configured to receive as input the drug's chemical structure and the specific patient's particular expressed protein. The system may be configured to predict binding between the drug and the protein and, if the drug's predicted binding affinity that particular patient's protein structure is too weak to be clinically effective, clinicians or practitioners may prevent that drug from being fruitlessly prescribed for the patient.

**Drug trial design:** This application generalizes the above personalized medicine use case to the case of patient populations. When the system can predict whether a drug will be effective for a particular patient phenotype, this information can be used to help design clinical trials. By excluding patients whose particular disease targets will not be sufficiently affected by a drug, a clinical trial can achieve statistical power using fewer patients. Fewer patients directly reduces the cost and complexity of clinical trials.

For this application, a user may segment the possible patient population into subpopulations that are characterized by the expression of different proteins (due to, for example, mutations or isoforms). The system may be configured to predict the binding strength of the drug candidate against the different protein types. If the predicted binding strength against a particular protein type indicates a necessary drug concentration that falls below the clinically-achievable in-patient concentration (as based on, for example, physical characterization in test tubes, animal models, or healthy volunteers), then the drug candidate is predicted to fail for that protein subpopulation. Patients with that protein may then be excluded from a drug trial.

**Agrochemical design:** In addition to pharmaceutical applications, the agrochemical industry uses binding prediction in the design of new pesticides. For example, one desideratum for pesticides is that they stop a single species of interest, without adversely impacting any other species. For ecological safety, a person could desire to kill a weevil without killing a bumblebee.

For this application, the user could input a set of protein structures, from the different species under consideration, into the system. A subset of proteins could be specified as the proteins against which to be active, while the rest would be specified as proteins against which the molecules should be inactive. As with previous use cases, some set of molecules (whether in existing databases or generated de novo) would be considered against each target, and the system would return the molecules with maximal effectiveness against the first group of proteins while avoiding the second.

**Materials science:** To predict the behavior and properties of new materials, it may be useful to analyze molecular interactions. For example, to study solvation, the user may input a repeated crystal structure of a given small molecule and assess the binding affinity of another instance of the small molecule on the crystal's surface. To study polymer strength, a set of polymer strands may be input analogously to a protein target structure, and an oligomer of the polymer may be input as a small molecule. Binding affinity between the polymer strands may therefore be predicted by the system.

In one specific example, the system may be used to predict the strength of a material such as Kevlar by, e.g., predicting the strength of hydrogen bonds and pi-bond stacks. So, binding affinity prediction as disclosed herein may be used to facilitate the development of improved materials such as Kevlar.

**Simulation:** Simulators often measure the binding affinity of a molecule to a protein, because the propensity of a molecule to stay in a region of the protein is correlates to its binding affinity there. An accurate description of the features governing binding could be used to identify regions and poses that have particularly high or low binding energy. The energetic description can be folded into Monte Carlo simulations to describe the motion of a molecule and the occupancy of the protein binding region. Similarly, stochastic simulators for studying and modeling systems biology could benefit from an accurate prediction of how small changes in molecule concentrations impact biological networks.

### General

It will be appreciated by those skilled in the art that other variations of the embodiments described herein may also be practiced without departing from the scope of the invention. Other modifications are therefore possible.

In further aspects, the disclosure provides systems, devices, methods, and computer programming products, including non-transient machine-readable instruction sets, for use in implementing such methods and enabling the functionality described previously.

Although the disclosure has been described and illustrated in exemplary forms with a certain degree of particularity, it is noted that the description and illustrations have been made by way of example only. Numerous changes in the details of construction and combination and arrangement of parts and steps may be made. Accordingly, such changes are intended to be included in the invention, the scope of which is defined by the claims.

Except to the extent explicitly stated or inherent within the processes described, including any optional steps or components thereof, no required order, sequence, or combination is intended or implied. As will be will be understood by those skilled in the relevant arts, with respect to both processes and any systems, devices, etc., described herein, a wide range of variations is possible, and even advantageous, in various circumstances, without departing from the scope of the invention, which is to be limited only by the claims.

## Claims

1. A system for predicting binding affinity of one molecule to one target protein, the system comprising:
at least one electronic datastore (12) configured to store records reflecting input data;
a data encoder module (14) configured to perform a method comprising:
constructing a data structure from the stored input data in the least one electronic datastore (12), the data structure providing a geometric representation of binding input features, wherein the constructing comprises:
augmenting the stored input data by generating additional instances of the stored input data through application of one or more pre-defined transformations of the stored input data wherein the one or more pre-defined transformations of the stored input data include a combination of rotation, translation, and mirror operators in any combination of the three X, Y, and Z planes of the stored input data thereby resulting in geometric data, and
truncating each instance of the stored input data in the geometric data to fit within a bounding box; and
populating the data structure by encoding data relating to the one molecule and the one target protein in the geometric data by, for each instance of the stored input data fitted to the bounding box in the geometric data:
partitioning the instance of the stored input data fitted to the bounding box in the geometric data into a corresponding voxel grid by transforming the instance of the input data fitted to the bounding box into a discrete set of regularly-spaced cubic voxels using a spacing that yields an integer number of voxels, wherein each respective voxel in the respective voxel grid comprises one or more input channels that encode a type of atom represented by the respective voxel in the respective voxel grid; and
a predictive model module (18) configured to:
apply a predictive model to the data structure to generate an indicator of a binding affinity for the one molecule to the one target protein represented by the data structure, wherein
the predictive model is a deep neural network or convolutional neural network comprising a convolutional filter layer comprising a set of function computation elements each of which takes as input a region of spatially contiguous voxels from a respective voxel grid in the data structure or convolutional functions thereof, and
the indicator of the binding affinity is an output of the predictive model.

2. The system of claim 1, wherein the geometric representation comprises a plurality of coordinates of atom centres.

3. The system of claim 1 or 2, wherein the geometric representation has a resolution between 0.1Å and 10Å.

4. The system of claim 3, wherein the geometric representation has a resolution of 1Å.

5. The system of any one of claims 1 to 4, wherein the predictive model module (18) is configured to train (28) the predictive model over time.

6. The system of any one of claims 1 to 5, wherein the records reflecting input data include records reflecting input data relating to at least one of atoms, ligand molecules, organic cofactors, inorganic cofactors, and proteins.

7. The system of any of claims 1 to 6, wherein the one or more input channels is a plurality of input channels and wherein the plurality of input channels further includes an input channel that specifies partial charge, polarizability, electronegativity, solvent accessible space, or electron density represented by the respective voxel in the respective voxel grid.

8. The system of any of claims 1 to 6, wherein the atom represented by a respective voxel in the respective voxel grid only partially occupies the respective voxel and the input channel in the one or more input channels that encodes the type of atom represented by the respective voxel is partially turned on proportional to the amount the atom represented by the respective voxel occupies the respective voxel.

9. The system of any of claims 1 to 6, wherein more than one atom type shares the same input channel in the one or more input channels.

10. A computer-implemented method for predicting binding affinity of one molecule to one target protein, the method comprising:
storing, in at least one electronic datastore (12), records reflecting input data;
constructing, at a data encoder module (14), a data structure from the stored input data in the least one electronic datastore (12), the data structure providing a geometric representation of binding input features, wherein the constructing comprises:
augmenting the stored input data by generating additional instances of the stored input data through application of one or more pre-defined transformations of the stored input data wherein the one or more pre-defined transformations of the stored input data include a combination of rotation, translation, and mirror operators in any combination of the three X, Y, and Z planes of the stored input data thereby resulting in geometric data, and
truncating each instance of the stored input data in the geometric data to fit within a bounding box; and
populating the data structure by encoding data relating to the one molecule and the one target protein in the geometric data by, for each instance of the stored input data fitted to the bounding box in the geometric data:
partitioning the instance of the stored input data fitted to the bounding box in the geometric data into a corresponding voxel grid by transforming the instance of the input data fitted to the bounding box into a discrete set of regularly-spaced cubic voxels using a spacing that yields an integer number of voxels, wherein each respective voxel in the respective voxel grid comprises one or more input channels that encode a type of atom represented by the respective voxel in the respective voxel grid; and
applying, at the at least one processor, a predictive model to the data structure to generate an indicator of a binding affinity for the one molecule to the one target protein represented by the data structure, wherein
the predictive model is a deep neural network or convolutional neural network comprising a convolutional filter layer comprising a set of function computation elements each of which takes as input a region of spatially contiguous voxels from a respective voxel grid in the data structure or convolutional functions thereof, and
the indicator of the binding affinity is an output of the predictive model.

11. The method of claim 10, wherein the geometric representation comprises a plurality of coordinates of atom centres.

12. The method of claim 10 or 11, wherein the geometric representation has a resolution between 0.1Å and 10Å.

13. The method of claim 12, wherein the geometric representation has a resolution of 1Å.

14. The method of any one of claims 10 to 13, further comprising training (28) the predictive model over time.

15. The method of any one of claims 10 to 14, wherein the records of input data include records reflecting input data relating to at least one of atoms, ligand molecules, organic cofactors, inorganic cofactors, proteins.

## Patentansprüche

1. Ein System zur Vorhersage der Bindeaffinität eines Moleküls an ein Zielprotein, das System umfassend:
mindestens ein elektronischer Datenspeicher (12), der konfiguriert ist, um Datensätze, die Input-Daten wiedergeben zu speichern;
ein Datenkodierungsmodul (14), das konfiguriert ist, um ein Verfahren auszuführen, umfassend:
Konstruieren einer Datenstruktur aus gespeicherten Input-Daten in dem mindestens einen Datenspeicher (12), wobei die Datenstruktur eine geometrische Wiedergabe der Binde-Input-Eigenschaften bereitstellt, wobei das Konstruieren umfasst:
Vergrößerung der gespeicherten Input-Daten durch Erzeugung zusätzlicher Instanzen der gespeicherten Input-Daten durch Anwendung einer oder mehrerer vor-definierter Transformationen der gespeicherten Input-Daten, wobei die eine oder mehreren vordefinierten Transformationen der gespeicherten Input-Daten eine Kombination von Rotation, Translation und Spiegeloperatoren in jeder Kombination der drei X, Y und Z Ebenen der gespeicherten Input-Daten umfasst, dadurch resultierend in geometrischen Daten, und
Kürzen jeder Instanz der gespeicherten Input-Daten in den geometrischen Daten, um in eine Begrenzungsrahmen zu passen; und
Einpflegen der Datenstruktur durch Kodierung der Daten, die zu einem Molekül und dem einen Zielprotein in den geometrischen Daten gehören, für jede Instanz der gespeicherten Input-Daten, die in den Begrenzungsrahmen in den geometrischen Daten passt:
Partitionierung der Instanz der gespeicherten Input-Daten, die an den Begrenzungsrahmen in den geometrischen Input-Daten angepasst wurde, in ein korrespondierendes Voxel-Gitter durch Transformation der Instanz der Input-Daten, die an den Begrenzungsrahmen angepasst wurden, in einen diskreten Satz von kubischen Voxeln mit gleichem Abstand durch Verwendung einer Verteilung, die eine gerade Anzahl an Voxeln erzielt, wobei jedes jeweilige Voxel in dem jeweiligen Voxelgitter ein oder mehrere Input-Kanäle umfasst, die einen Typ von Atom kodieren, der durch das jeweilige Voxel in dem jeweiligen Voxelgitter wiedergegeben ist; und
ein Vorhersagemodul (18) konfiguriert um:
Anwenden eines Vorhersagemodells auf die Datenstruktur, um einen Indikator für die Bindeaffinität für das eine Molekül an das eine Zielprotein, das durch die Datenstruktur wiedergegeben ist, zu erzeugen, wobei
das Vorhersagemodell ein tief-neuronales Netzwerk oder ein Faltungs-neuronales Netzwerk ist, das eine Faltungs-Filterschicht, umfassend einen Satz von Berechnungsfunktionselementen umfasst, wobei jedes davon als Input einen Bereich von räumlich zusammenhängenden Voxeln aus dem jeweiligen Voxelgitter in der Datenstruktur oder Faltungs-Funktionen davon verwendet, und
der Indikator der Bindeaffinität ein Output eines Vorhersagemodells ist.

2. Das System nach Anspruch 1, wobei die geometrische Wiedergabe eine Vielzahl von Koordinaten vom Atomzentren umfasst.

3. Das System nach Anspruch 1 oder 2, wobei die geometrische Wiedergabe eine Auflösung zwischen 0,1 Å und 10 Å hat.

4. Das System nach Anspruch 3, wobei die geometrische Wiedergabe eine Auflösung von 1 Å hat.

5. Das System nach einem der Ansprüche 1 bis 4, wobei das Vorhersagemodellmodul (18) konfiguriert ist, um das Vorhersagemodell über die Zeit zu trainieren (28).

6. Das System nach einem der Ansprüche 1 bis 5, wobei die Datensätze, die die Input-Daten wiedergeben, Datensätze einschließen, die Input-Daten wiedergeben, die sich auf mindestens eines von Atomen, Ligandmolekülen, organischen Kofaktoren, anorganischen Kofaktoren und Proteinen bezieht.

7. Das System nach einem der Ansprüche 1 bis 6, wobei der eine oder meherere Input-Kanäle eine Vielzahl von Input-Kanälen ist und wobei die Vielzahl von Input-Kanälen ferner einen Input-Kanal umfasst, der Teilladung, Polarisierbarkeit, Elektronegativität, Lösungsmittelzugangsbereich oder Elektronendichte spezifiziert, wiedergegeben durch das jeweilige Voxel in dem jeweiligen Voxelgitter.

8. Das System nach einem der Ansprüche 1 bis 6, wobei das Atom, das durch ein jeweiliges Voxel in dem jeweiligen Voxelgitter wiedergegeben wird, nur teilweise das jeweilige Voxel besetzt und der Input-Kanal in dem einen oder mehreren Input-Kanälen, der/ die den Typ von Atom in dem jeweiligen Voxel kodiert, teilweise aktiviert ist in Proportion zu dem Anteil mit dem das Atom, das durch das jeweilige Voxel wiedergegeben wird, das jeweilige Voxel besetzt.

9. Das System nach einem der Ansprüche 1 bis 6, wobei sich mehr als ein Atomtyp denselben Input-Kanal in dem einen oder mehreren Input-Kanälen teilt.

10. Ein Computer-implementiertes Verfahren zur Vorhersage der Bindeaffinität eines Moleküls an ein Zielprotein, das Verfahren umfassend:
Speichern, in mindestens einem elektronischen Datenspeicher (12), Datensätze, die Input-Daten wiedergeben;
Konstruieren, an einem Datenkodierungsmodul (14), eine Datenstruktur aus den gespeicherten Input-Daten in dem mindestens einen elektronischen Datenspeicher (12), wobei die Datenstruktur eine geometrische Wiedergabe der Bindungs-Input-Eigenschaften bereitstellt, wobei das Konstruieren umfasst:
Vergrößern der gespeicherten Input-Daten durch Erzeugung zusätzlicher Instanzen der gespeicherten Input-Daten durch Anwendung einer oder mehrerer vor-definierter Transformationen der gespeicherten Input-Daten, wobei die eine oder mehreren vordefinierten Transformationen der gespeicherten Input-Daten eine Kombination von Rotation, Translation, und Spiegeloperatoren in jeder Kombination der drei X, Y und Z Ebenen der gespeicherten Input-Daten, dadurch in geometrischen Daten resultierend, und
Verkürzung jeder Instanz der gespeicherten Input-Daten in den geometrischen Daten, um in den Begrenzungsrahmen zu passen, und
Einpflegen der Datenstruktur durch Kodierungsdaten, die zu dem einem Molekül und dem einen Zielprotein in den geometrischen Daten gehören für jede Instanz in den gespeicherten Input-Daten, die an den Begrenzungsrahmen in den geometrischen Daten angepasst ist:
Partitionierung der Instanz der gespeicherten Input-Daten, die an den Begrenzungsrahmen in den geometrischen Input-Daten angepasst wurde, in ein korrespondierendes Voxel-Gitter durch Transformation der Instanz der Input-Daten, die an den Begrenzungsrahmen angepasst wurden, in einen diskreten Satz von kubischen Voxeln mit gleichem Abstand durch Verwendung einer Verteilung, die eine gerade Anzahl an Voxeln erzielt, wobei jedes jeweilige Voxel in dem jeweiligen Voxelgitter ein oder mehrere Input-Kanäle umfasst, die einen Typ von Atom kodieren, der durch das jeweilige Voxel in dem jeweiligen Voxelgitter wiedergegeben ist; und
Anwenden, an dem mindestens einen Prozessor, ein Vorhersagemodell auf die Datenstruktur, um einen Indikator für die Bindeaffinität für das eine Molekül an das eine Zielprotein, das durch die Datenstruktur wiedergegeben ist, zu erzeugen, wobei
das Vorhersagemodell ein tief-neuronales Netzwerk oder ein Faltungs-neuronales Netzwerk ist, das eine Faltungs-Filterschicht, umfassend einen Satz von Berechnungsfunktionselementen umfasst, wobei jedes davon als Input einen Bereich von räumlich zusammenhängenden Voxeln aus dem jeweiligen Voxelgitter in der Datenstruktur oder Faltungs-Funktionen davon verwendet, und
der Indikator der Bindeaffinität ein Output eines Vorhersagemodells ist.

11. Das Verfahren nach Anspruch 10, wobei die geometrische Wiedergabe eine Vielzahl von Koordinaten vom Atomzentren umfasst.

12. Das Verfahren nach Anspruch 10 oder 11, wobei die geometrische Wiedergabe eine Auflösung zwischen 0,1Å und 10Å hat.

13. Das Verfahren nach Anspruch 12, wobei die geometrische Wiedergabe eine Auflösung von 1Å hat.

14. Das Verfahren nach einem der Ansprüche 10 bis 13, wobei das Vorhersagemodellmodul (18) konfiguriert ist, um das Vorhersagemodell über die Zeit zu trainieren (28).

15. Das Verfahren nach einem der Ansprüche 10 bis 14, wobei die Datensätze von Input-Daten Datensätze einschließen, die Input-Daten wiedergeben, die sich auf mindestens eines von Atomen, Ligandmolekülen, organischen Kofaktoren, anorganischen Kofaktoren und Proteinen beziehen.

## Revendications

1. Système pour prédire une affinité de liaison d'une molécule à une protéine cible, le système comprenant :
au moins une banque de données électronique (12) configurée pour stocker des enregistrements reflétant des données d'entrée ;
un module d'encodage de données (14) configuré pour effectuer un procédé comprenant :
la construction d'une structure de données à partir des données d'entrée stockées dans l'au moins une banque de données électronique (12), la structure de données fournissant une représentation géométrique de caractéristiques d'entrée de liaison, dans lequel la construction comprend :
l'augmentation des données d'entrée stockées en générant des cas supplémentaires des données d'entrée stockées par le biais de l'application d'une ou plusieurs transformations prédéfinies des données d'entrée stockées dans lequel les une ou plusieurs transformations prédéfinies des données d'entrée stockées incluent une combinaison d'opérateurs de rotation, translation et miroir dans n'importe quelle combinaison des trois plans X, Y et Z des données d'entrée stockées résultant ainsi en des données géométriques, et
le fait de tronquer chaque cas des données d'entrée stockées dans les données géométriques pour adaptation dans une boîte englobante ; et
le peuplement de la structure de données par des données d'encodage se rapportant à l'une molécule et à l'une protéine cible dans les données géométriques en, pour chaque cas des données d'entrée stockées adaptées à la boîte englobante dans les données géométriques :
divisant le cas des données d'entrée stockées adaptées dans la boîte englobante dans les données géométriques en une grille de voxels correspondante en transformant le cas des données d'entrée adaptées à la boîte englobante en un ensemble distinct de voxels cubiques régulièrement espacés à l'aide d'un espacement qui produit un nombre entier de voxels, dans lequel chaque voxel respectif de la grille de voxels respective comprend un ou plusieurs canaux d'entrée qui encodent un type d'atome représenté par le voxel respectif de la grille de voxels respective ; et
un module de modèle prédictif (18) configuré pour :
appliquer un modèle prédictif à la structure de données pour générer un indicateur d'une affinité de liaison pour l'une molécule vis-à-vis de la protéine cible représentée par la structure de données, dans lequel
le modèle prédictif est un réseau neuronal profond ou un réseau neuronal convolutif comprenant une couche filtrante convolutive comprenant un ensemble d'éléments de calcul de fonction dont chacun prend comme entrée une région de voxels spatialement contigus à partir d'une grille de voxels respective dans la structure de données ou des fonctions convolutives de celle-ci, et
l'indicateur de l'affinité de liaison est une sortie du modèle prédictif.

2. Système selon la revendication 1, dans lequel la représentation géométrique comprend une pluralité de coordonnées de centres d'atome.

3. Système selon la revendication 1 ou 2, dans lequel la représentation géométrique présente une résolution entre 0,1 Å et 10 Å.

4. Système selon la revendication 3, dans lequel la représentation géométrique présente une résolution de 1 Å.

5. Système selon l'une quelconque des revendications 1 à 4, dans lequel le module de modèle prédictif (18) est configuré pour former (28) le modèle prédictif avec le temps.

6. Système selon l'une quelconque des revendications 1 à 5, dans lequel les enregistrements reflétant des données d'entrée incluent des enregistrements reflétant des données d'entrée se rapportant à au moins l'un parmi des atomes, des molécules de ligand, des cofacteurs organiques, des cofacteurs inorganiques et des protéines.

7. Système selon l'une quelconque des revendications 1 à 6, dans lequel les un ou plusieurs canaux d'entrée sont une pluralité de canaux d'entrée et dans lequel la pluralité de canaux d'entrée incluent en outre un canal d'entrée qui spécifie une charge partielle, une polarisabilité, une électronégativité, un espace accessible au solvant ou une densité d'électrons, représenté par le voxel respectif dans la grille de voxels respective.

8. Système selon l'une quelconque des revendications 1 à 6, dans lequel l'atome représenté par un voxel respectif dans la grille de voxels respective occupe seulement partiellement le voxel respectif et le canal d'entrée dans les un ou plusieurs canaux d'entrée qui encode le type d'atome représenté par le voxel respectif est partiellement excité proportionnellement à la quantité que l'atome représenté par le voxel respectif occupe dans le voxel respectif.

9. Système selon l'une quelconque des revendications 1 à 6, dans lequel plus d'un type d'atome partage le même canal d'entrée dans les un ou plusieurs canaux d'entrée.

10. Procédé mis en œuvre par ordinateur pour prédire une affinité de liaison d'une molécule vis-à-vis d'une protéine cible, le procédé comprenant :
le stockage, dans au moins une banque de données électronique (12), d'enregistrements reflétant des données d'entrée ;
la construction, au niveau d'un module d'encodage de données (14), d'une structure de données à partir des données d'entrée stockées dans l'au moins une banque de données électronique (12), la structure de données fournissant une représentation géométrique de caractéristiques d'entrée de liaison, dans lequel la construction comprend :
l'augmentation des données d'entrée stockées en générant des cas supplémentaires des données d'entrée stockées par le biais de l'application d'une ou plusieurs transformations prédéfinies des données d'entrée stockées dans lequel les une ou plusieurs transformations prédéfinies des données d'entrée stockées incluent une combinaison d'opérateurs de rotation, translation et miroir dans n'importe quelle combinaison des trois plans X, Y et Z des données d'entrée stockées résultant ainsi en des données géométriques, et
le fait de tronquer chaque cas des données d'entrée stockées dans les données géométriques pour adaptation dans une boîte englobante ; et
le peuplement de la structure de données par des données d'encodage se rapportant à l'une molécule et à l'une protéine cible dans les données géométriques en, pour chaque cas des données d'entrée stockées adaptées à la boîte englobante dans les données géométriques :
divisant le cas des données d'entrée stockées adaptées dans la boîte englobante dans les données géométriques en une grille de voxels correspondante en transformant le cas des données d'entrée adaptées à la boîte englobante en un ensemble distinct de voxels cubiques régulièrement espacés à l'aide d'un espacement qui produit un nombre entier de voxels, dans lequel chaque voxel respectif de la grille de voxels respective comprend un ou plusieurs canaux d'entrée qui encodent un type d'atome représenté par le voxel respectif de la grille de voxels respective ; et
appliquant, au niveau de l'au moins un processeur, un modèle prédictif à la structure de données pour générer un indicateur d'une affinité de liaison pour l'une molécule vis-à-vis de la protéine cible représentée par la structure de données, dans lequel
le modèle prédictif est un réseau neuronal profond ou un réseau neuronal convolutif comprenant une couche filtrante convolutive comprenant un ensemble d'éléments de calcul de fonction dont chacun prend comme entrée une région de voxels spatialement contigus à partir d'une grille de voxels respective dans la structure de données ou des fonctions convolutives de celle-ci, et
l'indicateur de l'affinité de liaison est une sortie du modèle prédictif.

11. Procédé selon la revendication 10, dans lequel la représentation géométrique comprend une pluralité de coordonnées de centres d'atome.

12. Procédé selon la revendication 10 ou 11, dans lequel la représentation géométrique présente une résolution entre 0,1 Å et 10 Å.

13. Procédé selon la revendication 12, dans lequel la représentation géométrique présente une résolution de 1 Å.

14. Procédé selon l'une quelconque des revendications 10 à 13, comprenant en outre la formation (28) du modèle prédictif avec le temps.

15. Procédé selon l'une quelconque des revendications 10 à 14, dans lequel les enregistrements de données d'entrée incluent des enregistrements reflétant des données d'entrée se rapportant à au moins l'un parmi des atomes, des molécules de ligand, des cofacteurs organiques, des cofacteurs inorganiques, des protéines.
